(19) Europäisches Patentamt
European Patent Office
Office européen des brevets

(11) Veröffentlichungsnummer: **0 006 539**
**A1**

(12) # EUROPÄISCHE PATENTANMELDUNG

(21) Anmeldenummer: **79101940.9**

(22) Anmeldetag: **15.06.79**

(51) Int. Cl.³: **C 07 C 69/66,** C 07 C 67/22

(30) Priorität: **26.06.78 DE 2828011**
**26.06.78 DE 2827977**

(43) Veröffentlichungstag der Anmeldung: **09.01.80**
**Patentblatt 80/1**

(84) Benannte Vertragsstaaten: **BE CH DE FR GB IT NL**

(71) Anmelder: **BAYER Aktiengesellschaft, Zentralbereich Patente,Marken und Lizenzen Bayerwerk, D-5090 Leverkusen 1 (DE)**

(72) Erfinder: **Schwarz, Hans-Helmut, Dr., Ratherstrasse 90, D-4150 Krefeld (DE)**
Erfinder: **Krätzer, Hans, Dr., Am Acker 17, D-5600 Wuppertal 1 (DE)**
Erfinder: **Singer, Rolf-Jürgen, Dr., Platzhoffstrasse 55, D-5600 Wuppertal 1 (DE)**

(54) **Verfahren zur Herstellung von Mandelsäureestern.**

(57) Die Erfindung betrifft ein neues einstufiges Verfahren zur Herstellung von Mandelsäureestern der allgemeinen Formel

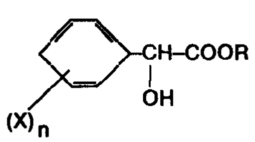

(I)

in welcher
R für Alkyl steht,
X für Alkyl, Aryl, Hydroxy, Alkoxy, Halogen, Halogenalkyl und/oder Nitro steht, und
n für eine ganze Zahl von 0 bis 3 steht,
durch Umsetzung von Mandelsäurenitrilen (Benzaldehydcyanhydrinen), Alkoholen und Chlorwasserstoff, wobei man gewünschtenfalls den Nitrilstickstoff gleichzeitig in Form von festem Ammoniumchlorid gewinnen kann. Das Verfahren ist dadurch gekennzeichnet, daß man die entsprechenden Mandelsäurenitrile pro Mol mit 3 bis 20 Mol eines Alkohols der Formel R-OH und entweder mit 1,8 bis 3 Mol Chlorwasserstoff oder unter Zusatz von bis zu 1 Mol Wasser mit 1,2 bis 2,4 Mol Chlorwasserstoff bei Temperaturen zwischen 40 und 160° C umsetzt und nach einer Reaktionszeit von zirka 3 bis 10 Stunden.

a) in üblicher Weise aufarbeitet und die Produkte isoliert oder

b) — für den Fall, daß man den Nitrilstickstoff in Form von festem Ammoniumchlorid zu gewinnen wünscht —, überschüssigen Chlorwasserstoff mit gasförmigem Ammoniak neutralisiert, einen aromatischen, gegebenenfalls chlorierten Kohlenwasserstoff zusetzt und zur Aufarbeitung den überschüssigen Alkohol abdestilliert, nach Abkühlen das ausgeschiedene kristalline Ammoniumchlorid abfiltriert und den Mandelsäureester aus dem organischen Filtrat durch Destillation isoliert.

Mie Mandelsäureester dienen als organische Zwischenprodukte, z. B. für die Herstellung von herbiziden Wirkstoffen.

EP 0 006 539 A1

0006539

BAYER AKTIENGESELLSCHAFT
Zentralbereich
Patente, Marken und Lizenzen

5090 Leverkusen, Bayerwerk
Bi-kü
IVa/ZP

## Verfahren zur Herstellung von Mandelsäureestern

Die vorliegende Erfindung betrifft ein einstufiges Verfahren zur Herstellung von Mandelsäureestern, ausgehend von Mandelsäurenitrilen (Benzaldehyd-cyanhydrinen), wobei gegebenenfalls der Nitrilstickstoff gleichzeitig in Form von festem Ammoniumchlorid gewonnen werden kann. Mandelsäureester können als Zwischenprodukte, z.B. zur Herstellung von herbiziden Wirkstoffe, verwendet werden.

Die aus der Literatur bekannten Synthesen von Mandelsäureestern aus den Nitrilen werden in der Regel zweistufig über die Iminoäther-hydrochloride durchgeführt und liefern Ausbeuten von ca. 70 % (vgl. Chem. Abstr. Vol. 70, 11 290r (1969); vgl. ferner Houben-Weyl, Methoden der organischen Chemie, 4. Auflage, Band VIII, S. 539).

In der Literatur ist auch bereits eine einstufige Umsetzung von Mandelsäurenitril zu Mandelsäureäthylester beschrieben worden; hierbei wurde jedoch nur eine Ausbeute von ca. 50 % erzielt (J. prakt. Chem. (2) Bd.150, S. 90 (1938)).

Ein besonderer Nachteil dieser vorbekannten Verfahren besteht ferner darin, daß der in der Nitrilgruppe vorhandene Stickstoff nicht verwertet wird, sondern bei der Aufarbeitung verloren geht bzw. nur mit außerordentlichem Aufwand in Form von Ammonsalzen isoliert werden kann.

Es wurde nun gefunden, daß man Mandelsäureester der Formel

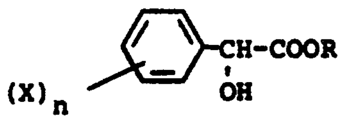

(I)

in welcher

R für Alkyl steht,

X für Alkyl, Aryl, Hydroxy, Alkoxy, Halogen, Halogenalkyl und/oder Nitro steht und

n für eine ganze Zahl von 0 bis 3 steht,

durch Umsetzung von Mandelsäurenitrilen (Benzaldehydcyanhydrinen), Alkoholen und Chlorwasserstoff in sehr hoher Ausbeute und mit sehr guter Reinheit erhält, wobei man gewünschtenfalls den Nitrilstickstoff gleichzeitig in Form von festem Ammoniumchlorid gewinnen kann, wenn man Mandelsäurenitrile der Formel

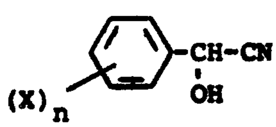

(II)

in welcher

X und n die oben angegebene Bedeutung haben,

pro Mol mit 3 bis 20 Mol eines Alkohols der Formel

R-OH (III)

in welcher
R die oben angegebene Bedeutung hat,

und entweder mit 1,8 bis 3 Mol Chlorwasserstoff oder unter Zusatz von bis zu 1 Mol Wasser mit 1,2 bis 2,4 Mol Chlorwasserstoff bei Temperaturen zwischen 40 und 160°C umsetzt und nach einer Reaktionszeit von ca. 3 bis 10 Stunden

a) in üblicher Weise aufarbeitet und die Produkte isoliert oder
b) - für den Fall, daß man den Nitrilstickstoff in Form von festem Ammoniumchlorid zu gewinnen wünscht, -

überschüssigen Chlorwasserstoff mit gasförmigem Ammoniak neutralisiert, einen aromatischen, gegebenenfalls chlorierten Kohlenwasserstoff zusetzt und zur Aufarbeitung den überschüssigen Alkohol abdestilliert, nach Abkühlen das ausgeschiedene kristalline Ammoniumchlorid abfiltriert und den Mandelsäureester aus dem organischen Filtrat durch Destillation isoliert.

Es ist im Hinblick auf den Stand der Technik als ausgesprochen überraschend zu bezeichnen, daß es unter den Bedingungen des erfindungsgemäßen Verfahrens gelingt, Mandelsäurenitrile auf technisch einfache Weise mit hoher Ausbeute in sehr reine Mandelsäureester zu überführen, wobei sich gewünschtenfalls gleichzeitig festes Ammoniumchlorid gewinnen läßt.

Das erfindungsgemäße Verfahren weist eine Reihe von Vorteilen auf. Insbesondere liefert das neue, einstufige Verfahren Ausbeuten an Mandelsäureestern (über 90 % der Theorie), die weit über die nach vergleichbaren bekannten Verfahren erzielbaren Ausbeuten (50 bis 70 %) hinausgehen. Das neue Verfahren zeichnet sich gegenüber den vorbekannten Herstellungsmethoden wesentlich dadurch aus,

daß ein Überschuß an Chlorwasserstoff pro Mol Mandelsäurenitril (II) bei erhöhter Temperatur in Gegenwart überschüssigen Alkohols (III) eingesetzt wird. Durch diese Maßnahmen lassen sich die sonst eintretenden, ausbeutevermindernden Nebenreaktionen weitgehend unterdrücken. Der Anteil an Nebenprodukten ist infolgedessen minimal, und die durch Vakuumdestillation isolierten Mandelsäureester können ohne weitere Reinigung unmittelbar für anschließende Umsetzungen eingesetzt werden.

Ein zusätzlicher Vorteil des neuen Verfahrens besteht darin, daß es bei Aufarbeitung nach Variante (b) auf technisch einfache Weise gelingt, den Stickstoff der Nitrilgruppe direkt in Form von festem, kristallinen Ammoniumchlorid zu gewinnen. Demgegenüber entstand bei den bisher bekannten Verfahren dieser Art stets eine wäßrige Salzlösung, aus der das Ammoniumchlorid nur durch technisch aufwendige und energieverbrauchende Operationen gewonnen werden konnte; da man hierauf meist verzichten muß, geht das Ammoniumchlorid bisher praktisch immer verloren.

Darüber hinaus läßt sich der Einsatzüberschuß an Alkohol und Chlorwasserstoff zur Bildung von Alkylchloriden ausnutzen, welche aufgrund ihrer relativ niederen Siedepunkte leicht aus dem Reaktionsgemisch zu isolieren sind.

Verwendet man Mandelsäurenitril (Benzaldehyd-cyanhydrin), Äthylalkohol und Chlorwasserstoff als Ausgangsstoffe, so kann der Reaktionsablauf im wesentlichen durch das folgende (stöchiometrische) Formelschema wiedergegeben werden, wobei der erfindungsgemäß notwendige Einsatzüberschuß an Äthylalkohol und Chlorwasserstoff allerdings nicht berücksichtigt ist:

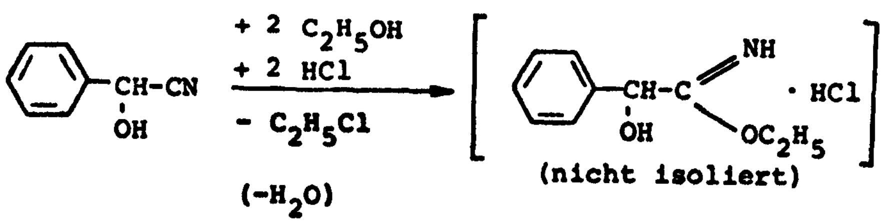


Aufarbeitung nach Variante (a):

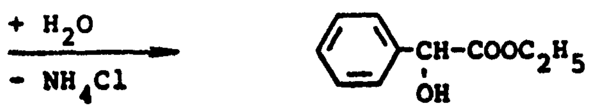


Aufarbeitung nach Variante (b):

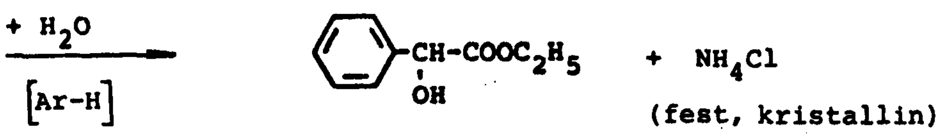


"Ar-H" bezeichnet einen aromatischen, gegebenenfalls chlorierten Kohlenwasserstoff.

Die als Ausgangsstoffe zu verwendenden Mandelsäurenitrile (Benzaldehyd-cyanhydrine) sind durch die Formel (II) allgemein definiert. In dieser Formel steht der Index n vorzugsweise für 0 und 1. Der Rest X steht vorzugsweise für Alkyl mit 1 bis 4 C-Atomen, Phenyl, Hydroxy, Alkoxy mit 1 bis 2 C-Atomen, Chlor, Halogenmethyl, insbesondere Trifluormethyl sowie für die Nitrogruppe. Als Beispiele seien genannt:
Benzaldehyd-cyanhydrin, 4-Methylbenzaldehyd-cyanhydrin, 3-Äthylbenzaldehyd-cyanhydrin, 4-tert.-Butylbenzaldehyd-

cyanhydrin, 4-Phenylbenzaldehyd-cyanhydrin, 3-Hydroxy- und 4-Hydroxybenzaldehyd-cyanhydrin, Anisaldehydcyanhydrin, 3-Chlorbenzaldehyd-cyanhydrin, 4-Trifluormethylbenzaldehyd-cyanhydrin, 3-Nitro- und 4-Nitrobenzaldehyd-cyanhydrin.

Die erfindungsgemäß zu verwendenden Mandelsäurenitrile (II) sind großenteils bekannt und können in bekannter Weise durch Umsetzung der entsprechenden Benzaldehyde mit Blausäure hergestellt werden (vgl. z.B. Houben-Weyl, Methoden der Organ. Chemie, 4. Auflage, Band VIII, S. 274 ff.)

Die weiterhin aus Ausgangsstoffe zu verwendenden Alkohole sind durch die Formel (III) allgemein definiert. Hier steht R vorzugsweise für Alkyl mit 1 bis 6 C-Atomen. Als Beispiele seien genannt:
Methanol, Äthanol, n-Propanol, Isopropanol, n-Butanol, Isobutanol, n-Hexanol.

Die jeweils im Überschuß eingesetzten Alkohole (III) dienen zugleich als Verdünnungsmittel.

Das erfindungsgemäße Verfahren wird im Temperaturbereich von 40 bis 160$^{o}$C, vorzugsweise von 45 bis 100$^{o}$C durchgeführt. Bei Herstellung der Methyl- und Äthylester wählt man als Reaktionstemperatur am zweckmäßigsten die Siedetemperatur des Methyl- bzw. Äthylalkohols.

Bei der Durchführung des erfindungsgemäßen Verfahrens setzt man auf 1 Mol Mandelsäurenitril der Formel (II) einen Überschuß von 3 bis 20 Mol, vorzugsweise von 5 bis 16 Mol, Alkohol (III) ein. Arbeitet man ohne Zusatz von Wasser, so setzt man auf 1 Mol Mandelsäurenitril (II) ferner 1,8 bis 3 Mol, vorzugsweise 2,1 bis 2,5 Mol, Chlorwasserstoff ein.

Arbeitet man unter Zusatz von Wasser, so setzt man auf 1 Mol Mandelsäurenitril (II) bis zu 1 Mol, vorzugsweise 0,4 bis 0,8 Mol Wasser und 1,2 bis 2,4 Mol, vorzugsweise 1,3 bis 2,2 Mol Chlorwasserstoff ein. (Dieser Wasserzusatz bewirkt, daß ein Teil des zur Esterbildung erforderlichen Wassers nicht durch die Reaktion zwischen Alkohol und Chlorwasserstoff gebildet werden muß; man kann deshalb den Überschuß an Chlorwasserstoff verringern, gewinnt dann allerdings auch entsprechend weniger Alkylchlorid.) Die Reaktionszeiten betragen im allgemeinen etwa 3 bis 10 Stunden.

Verzichtet man auf die Isolierung von festem Ammoniumchlorid, so erfolgt die Aufarbeitung und Isolierung der Mandelsäuren (I) gemäß Varinate (a) in allgemein üblicher Weise.

Die nachfolgenden Angaben beziehen sich auf die Aufarbeitung des Reaktionsgemisches nach Variante (b). Will man das gebildete Ammoniumchlorid in fester Form isolieren, so wird nach beendeter Reaktion noch freier überschüssiger Chlorwasserstoff mit gasförmigem Ammoniak neutralisiert, zweckmäßigerweise bis zu einem pH-Wert zwischen 3 und 4, vorzugsweise zwischen 3,3 und 3,9. Dann wird dem Reaktionsgemisch ein aromatischer gegebenenfalls chlorierter Kohlenwasserstoff zugesetzt, um das gebildete Ammoniumchlorid vollständig in fester Form abzuscheiden. Der Kohlenwasserstoff bzw. Chlorkohlenwasserstoff kann als Flüssigkeit oder vorteilhafter als Dampf zugefügt werden, so daß gleichzeitig ein Teil der zur Verdampfung des überschüssigen Alkohols notwendigen Wärmemenge zugeführt wird.

Auf 1 Gewichtsteil Mandelsäurenitril werden im allgemeinen 1 bis 20 Gewichtsteile, vorzugsweise 2 bis 15

Gewichtsteile, des aromatischen Kohlenwasserstoffes bzw. Chlorkohlenwasserstoffes eingesetzt.

Als aromatische Kohlenwasserstoffe bzw. Chlorkohlenwasserstoffe sind solche Verbindungen geeignet, deren Siedepunkte höher als der des verwendeten Alkohols liegen, wie z.B. Benzol, Toluol, Cumol, Xylol, Chlorbenzol, Dichlorbenzol, Chlortoluol.

Gleichzeitig oder anschließend an die Zugabe des aromatischen Kohlenwasserstoffs bzw. Chlorkohlenwasserstoffs zur Reaktionsmischung führt man dem System Wärme zu, um den Alkohol abzudestillieren. Dieser Vorgang kann bei erhöhtem Druck, Normaldruck oder Unterdruck durchgeführt werden.

Das gesamte Verfahren kann in diskontinuierlichen Ansätzen oder in kontinuierlicher Fahrweise durchgeführt werden. Bei einer kontinuierlichen Reaktionsführung ist es besonders empfehlenswert, das Reaktionsgemisch in eine Destillationskolonne einzuspeisen und entgegen diesem Produktstrom den Dampf des aromatischen Kohlenwasserstoffs strömen zu lassen.

Die Abtrennung des Alkohols aus dem Reaktionsgemisch soll soweit erfolgen, daß der Gehalt an dieser Verbindung in der Mischung unter 0,3 % beträgt.

Bei der Durchführung dieses Prozesses entsteht das Ammoniumchlorid in kristalliner fester Form. Die Größe dieser Kristalle hängt weitgehend von der Geschwindigkeit ab, mit welcher der Alkohol aus dem Reaktionsgemisch entfernt wird. Je schneller dies verläuft, um so kleiner sind die entstehenden Ammoniumchloridkristalle.

Es ist daher vorteilhaft, die Hauptmenge des Alkohols während eines Zeitraumes von etwa 0,5-5 Stunden durch Destillation abzutrennen. Die Beseitigung der Restmengen kann dann schnell erfolgen, ohne daß die mittlere Korngröße entscheidend verkleinert wird.

Das feste Ammoniumchlorid kann durch Filtration oder Zentrifugieren einfach von der Lösung des Mandelsäureesters abgetrennt werden. Es wird mit dem aromatischen Kohlenwasserstoff bzw. Chlorkohlenwasserstoff gewaschen und anschließend getrocknet. Die Waschflüssigkeit wird in den Prozess zurückgeführt.

Die gebildeten Mandelsäureester werden aus dem Filtrat in üblicher Weise durch Destillation isoliert.

---

Die erfindungsgemäß herstellbaren Mandelsäureester der Formel (I) sind großenteils bekannt; sie können z.B. als Zwischenprodukte zur Herstellung von Pharmazeutika und Schädlingsbekämpfungsmitteln, insbesondere von Herbiziden, verwendet werden. Beispielsweise erhält man aus dem Mandelsäuremethylester (1) nach folgendem Reaktionsschema die herbizid wirksame Verbindung 4-Amino-3-methyl-6-phenyl-1,2,4-triazin-5(4H)-on (vgl. DE-OS 2 224 161):

1. Stufe

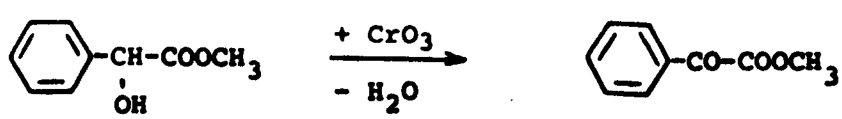


(1) (A)

2. Stufe

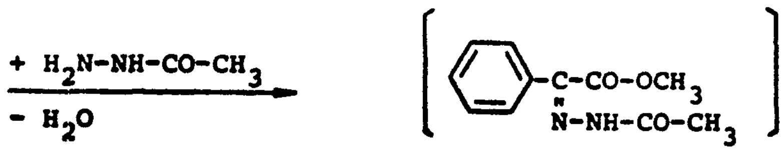


(B)

wird nicht isoliert

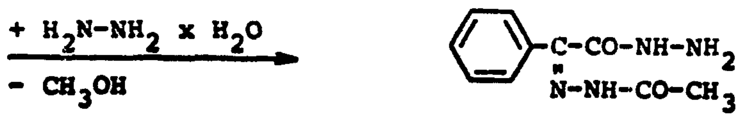


(C)

3. Stufe

(C)

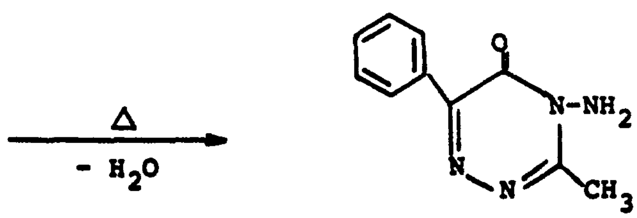


(D)

1. Stufe

0,7 Mol Mandelsäuremethylester (1) werden in 200 g Eisessig gelöst. Diese Lösung wird zu einer Mischung aus 700 g Eisessig und 0,9 Mol Chromtrioxid ($CrO_3$) innerhalb von 30 Minuten bei 50°C zugetropft. Nach 2-stündiger Rührzeit bei dieser Temperatur wird die Essigsäure weitgehend im Vakuum bei 20 mbar abdestilliert. Der Destillationsrückstand wird mit 1126 g Wasser vermischt. Die organische Phase wird abgetrennt, und die

wäßrige Phase wird 3mal mit je 100 g Methylisobutylketon extrahiert. Die vereinigten organischen Phasen werden im Vakuum fraktioniert. Der Hauptlauf (102,5 g) siedet bei 16 mbar zwischen 120 und 126°C. Die Ausbeute an Phenylglyoxylsäuremethylester (A) beträgt 89,5 % d.Th.

2. Stufe

1 Mol Phenylglyoxylsäuremethylester (A) und 1 Mol Acetylhydrazin werden in 780 g Isopropanol 5 Stunden unter Rückfluß erhitzt. Als Katalysator wird etwas p-Toluolsulfonsäure zugesetzt. Das Hydrazon (B) fällt als Isomerengemisch an, wobei ein Isomeres kristallisiert. Deshalb wird weitergearbeitet ohne das Hydrazon zu isolieren. Zu dieser Reaktionslösung wird, nachdem der Ester verbraucht ist, Hydrazinhydrat gegeben und unter Rühren 5 Stunden bei 20°C gehalten. Danach wird das Reaktionsgemisch auf 0 bis 5°C abgekühlt und abgesaugt. Die Ausbeute an reinem Hydrazid (C) beträgt 85 %.

3. Stufe

1 Mol Hydrazid (C) wird mit 1000 g Isopropanol versetzt und 24 Stunden bei 100°C im geschlossenen Kessel (1-2 bar Überdruck) gerührt. Anschließend wird bei -5°C abgesaugt. In 84 %iger Ausbeute erhält man das 4-Amino-3-methyl-6-phenyl-1,2,4-triazin-5(4H)-on (D) vom Schmelzpunkt 167-169°C.

Das erfindungsgemäße Verfahren wird durch die nachfolgenden Herstellungsbeispiele veranschaulicht.

Herstellungsbeispiele

Beispiel 1 (Aufarbeitung nach Variante (a))

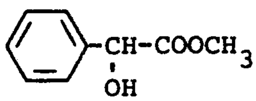

(1)

In einem 3-Halskolben mit Rührer, Rückflußkühler und Tropftrichter wurden 16,7 Mol Methylalkohol vorgelegt und auf 50°C erwärmt. Dann wurden möglichst schnell 4 Mol Benzaldehyd-cyanhydrin und eine Lösung von 7,2 Mol HCl in 11,3 Mol Methylalkohol zugetropft.

Die Reaktionsmischung wurde anschließend 6 Stunden auf 50°C erwärmt. Während dieser Zeit entwichen durch den Kühler 3,45 Mol Methylchlorid ($CH_3Cl$), die in einer Kühlfalle kondensiert werden können. In der Lösung waren noch 0,15 Mol Methylchlorid nachweisbar.

Nach beendeter Reaktion wurde überschüssiger Chlorwasserstoff durch Neutralisation mit $NH_3$ in Ammoniumchlorid überführt. Das Reaktionsgemisch wurde mit Wasser verdünnt, mit Äther mehrfach extrahiert. Der Ätherextrakt wurde destilliert. Bei 26,6 mbar und 138-140°C destillierten 598 g Mandelsäuremethylester. Dies entspricht einer Ausbeute von 90,1 %, bezogen auf Benzaldehyd-cyanhydrin.

Beispiel 2 (Aufarbeitung nach Variante (a))

In eine Lösung von 1 Mol Benzaldehyd-cyanhydrin in 15,5 Mol Methylalkohol wurden im Laufe von 2 Stunden bei 66°C 2,7 Mol HCl eingeleitet. Anschließend wurde noch weitere 6 Stunden unter Rückfluß gekocht (66°C).

Durch Analyse wurde die Bildung von insgesamt 1,2 Mol Methylchlorid nachgewiesen. Die Aufarbeitung des Reaktionsgemisches nach Beispiel 1 ergab Mandelsäuremethylester (1) in einer Ausbeute von 95 %.

Beispiel 3 (Aufarbeitung nach Variante (a))

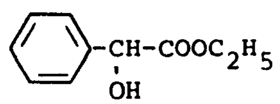

(2)

In einem 3-Halskolben mit Rührer, Rückflußkühler und Tropftrichter wurden 5 Mol Äthylalkohol vorgelegt und auf 55°C erwärmt. Dann wurde eine Lösung von 2 Mol Benzaldehyd-cyanhydrin in 5 Mol Äthylalkohol und eine Lösung von 5,4 Mol HCl in 21 Mol Äthylalkohol so zugetropft, daß die Temperatur von 55°-60° gehalten wurde. Nach beendeter Zugabe wurde noch 6 Stunden unter Rückfluß gekocht (ca. 80°C).

Eine gaschromatographische Analyse des Reaktionsgemisches ergab eine Ausbeute von 95,1 % Mandelsäureäthylester, bezogen auf eingesetztes Benzaldehyd-cyanhydrin. Gleichzeitig wurden 2,1 Mol Äthylchlorid nachgewiesen.

Die Aufarbeitung und Isolierung des Mandelsäureäthylesters wurde wie in Beispiel 1 beschrieben durchgeführt.

**Beispiel 4** (Aufarbeitung nach Variante (a))

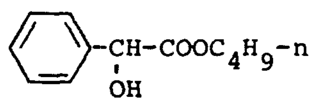

(3)

Nach der Methode von Beispiel 1 wurden 2 Mol Benzaldehyd-cyanhydrin, 31 Mol n-Butanol und 5,4 Mol HCl zur Reaktion gebracht. Die Reaktionstemperatur wurde 10 Stunden bei 65°C gehalten.

Nach dieser Reaktionszeit wurde im Reaktionsgemisch durch GC-Analyse Mandelsäurebutylester in einer Ausbeute von 97 %, bezogen auf eingesetztes Benzaldehyd-cyanhydrin, nachgewiesen.

Die Aufarbeitung und Isolierung des Mandelsäure-n-butylesters wurde wie in Beispiel 1 beschrieben durchgeführt.

**Beispiel 5** (Aufarbeitung nach Variante (a))

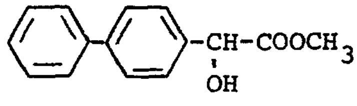

(4)

Nach der Methode von Beispiel 1 wurden 0,23 Mol p-Phenylbenzaldehyd-cyanhydrin , 3,9 Mol Methanol und 0,55 Mol HCl zur Reaktion gebracht.

Nach 7-stündiger Kochzeit wurde das Reaktionsgemisch mit Wasser versetzt, mit Ammoniak neutralisiert, und die wäßrige Phase wurde erschöpfend mit Chlorbenzol (900 g) extrahiert.

Die organische Phase wurde eingedampft. Nachdem 430 g Chlorbenzol abdestilliert waren, wurde von einer kleinen Menge eines Rückstandes (1,3 g) abfiltriert. Das Filtrat wurde i.V. weiter bis zur Trockne eingedampft.

Es resultierte ein Rückstand von p-Phenylmandelsäuremethylester (52,5 g) mit einer Esterzahl von 231-234 (Theorie 231,6). Das entspricht einer Ausbeute von 93 %, bezogen auf eingesetztes p-Phenylbenzaldehyd-cyanhydrin.

Beispiel 6 (Aufarbeitung nach Variante (a))

In eine Mischung aus 1 Mol Benzaldehyd-cyanhydrin, 0,5 Mol Wasser und 15,5 Mol Methanol wurden bei ca. 65°C 1,3 Mol HCl eingeleitet. Anschließend wurde noch weitere 4 Stunden unter Rückfluß gekocht.

Durch Analyse wurde die Bildung von insgesamt 0,28 Mol Methylchlorid nachgewiesen. Die Aufarbeitung des Reaktionsgemisches nach Beispiel 1 ergab Mandelsäuremethylester (1) in einer Ausbeute von 97 % d.Th.

Beispiel 7 (Aufarbeitung nach Varinate (b))

In einem 3-Halskolben mit Rührer, Rückflußkühler und Tropftrichter wurden 6 Mol Methanol vorgelegt und auf 50°C erwärmt. Dann wurde möglichst schnell 1 Mol Benzaldehyd-cyanhydrin und eine Lösung von 2,5 Mol Chlorwasserstoff in 4 Mol Methanol zugetropft.

Die Reaktionsmischung wurde anschließend 6,5 Stunden bei 65°C unter Rückfluß erhitzt. Während dieser Zeit entwichen durch den Kühler 0,95 Mol Methylchlorid ($CH_3Cl$), die in einer Kühlfalle kondensiert werden können.

Anschließend wurde das Reaktionsgemisch mit gasförmigem $NH_3$ auf einen pH-Wert von 3,5 eingestellt und mit 700 g Toluol versetzt. Dann wurde bei Normaldruck ein Gemisch von Methanol und Toluol abdestilliert. Nachdem im Laufe von 3 Stunden 500 g Destillat angefallen waren, wurden dem Ansatz nochmals 500 g Toluol zugemischt. Anschließend wurde noch so lange destilliert, bis eine GC-Analyse des Kolbeninhaltes einen Gehalt von 0,1 % Methanol ergab. Das Reaktionsgemisch wurde schließlich abgekühlt und filtriert. Der Filterrückstand wurde mit 100 g Toluol gewaschen und dann getrocknet; er bestand aus 72 g festem Ammoniumchlorid.

Das Filtrat und die zum Waschen des Ammoniumchlorids benutzte Toluolmenge wurden vereinigt und durch Destillation aufgearbeitet.
Man erhielt 154 g Mandelsäuremethylester (Ausbeute 93,6 % der Theorie); Siedepunkt 123,5 - 124°C bei 13.4 mbar.

Beispiel 8 (Aufarbeitung nach Variante (b))

Unter den Bedingungen des Beispiels 1 wurden 5 Mol Benzaldehyd-cyanhydrin, 55 Mol Methanol und 11 Mol Chlorwasserstoff umgesetzt. Während der Reaktion entwichen 4,8 Mol Methylchlorid. Der Reaktionsansatz wurde nach Neutralisation mit gasförmigem $NH_3$ (pH = 3,85 ) mit 2 kg Chlorbenzol versetzt. Im Laufe von 5 Stunden wurde bei Normaldruck das Methanol abdestilliert, bis die Temperatur der destillierenden Dämpfe die Siedetemperatur des Chlorbenzols erreicht hatten.

Die Reaktionsmischung wurde abgekühlt und filtriert. Der Rückstand wurde mit 500 g Chlorbenzol gewaschen und anschließend getrockent. Es wurden 307 g festes Ammoniumchlorid gewonnen, dessen mittlere Korngröße 0,15 mm betrug.

Das Filtrat und die zum Waschen des Ammoniumchlorids benutzte Chlorbenzolmenge wurden vereinigt und durch Destillation aufgearbeitet.

Man erhielt 786 g Mandelsäuremethylester (Ausbeute 95 % der Theorie); Siedepunkt 123,5 - 124°C bei 13,4 mbar.

Beispiel 9 (Aufarbeitung nach Variante (b))

In ein Gemisch aus 1 Mol Benzaldehyd-cyanhydrin, 0,5 Mol Wasser und 16 Mol Methanol wurden im Laufe von 45 Minuten bei ca. 50°C 1,3 Mol HCl-Gas eingeleitet. Das Reaktionsgemisch wurde anschließend noch 9 Stunden unter Rückfluß gekocht. Dabei entwichen durch den Kühler 0,21 Mol Methylchlorid, die in einer Kühlfalle kondensiert wurden.

Das Reaktionsgemisch wurde anschließend durch Einleiten von gasförmigem $NH_3$ neutralisiert (pH = 3,6) und mit 6,6 Mol Chlorbenzol versetzt. Dann wurde destilliert, bis der Reaktionssumpf kein Methanol mehr enthielt. Man kühlte auf Raumtemperatur ab und filtrierte das ausgeschiedene Ammoniumchlorid ab und wusch es mit Chlorbenzol. Nach dem Trocknen erhielt man 61 g festes Ammoniumchlorid.

Im methanolischen Destillat waren analytisch noch 0,07 Mol Methylchlorid nachzuweisen. Das Filtrat und die zum Waschen des Ammoniumchlorids verwendete Chlorbenzolmenge wurden vereinigt und wiederum destillativ aufgearbeitet.

Man erhielt 150 g Mandelsäuremethylester (1) (Ausbeute 91 % der Theorie); Siedepunkt 123,5 bis 124°C bei 13,4 mbar.

Patentansprüche

1. Verfahren zur Herstellung von Mandelsäureestern der Formel

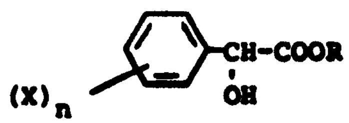

(I)

in welcher

R für Alkyl steht,

X für Alkyl, Aryl, Hydroxy, Alkoxy, Halogen, Halogenalkyl und/oder Nitro steht und

n für eine ganze Zahl von 0 bis 3 steht,

durch Umsetzung von Mandelsäurenitrilen mit Alkoholen und Chlorwasserstoff, bei welchem gegebenenfalls der Nitrilstickstoff gleichzeitig in Form von festem Ammoniumchlorid gewonnen werden kann, dadurch gekennzeichnet, daß man in einstufiger Verfahrensweise Mandelsäurenitrile der Formel

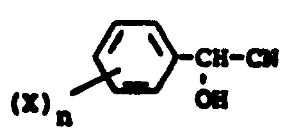

(II)

in welcher

X und n die oben angegebene Bedeutung haben,

pro Mol mit 3 bis 20 Mol eines Alkohols der Formel

R-OH (III)

in welcher
R die oben angegebene Bedeutung hat,

und entweder mit 1,8 bis 3 Mol Chlorwasserstoff oder unter Zusatz von bis zu 1 Mol Wasser mit 1,2 bis 2,4 Mol Chlorwasserstoff bei Temperaturen zwischen 40 und 160°C umsetzt und nach einer Reaktionszeit von ca. 3 bis 10 Stunden

(a) in üblicher Weise aufarbeitet und die Produkte isoliert oder

(b) - für den Fall, daß man den Nitrilstickstoff in Form von festem Ammoniumchlorid zu gewinnen wünscht, - überschüssigen Chlorwasserstoff mit gasförmigem Ammoniak neutralisiert, einen aromatischen, gegebenenfalls chlorierten Kohlenwasserstoff zusetzt und zur Aufarbeitung den überschüssigen Alkohol abdestilliert, nach Abkühlen das ausgeschiedene kristalline Ammoniumchlorid abfiltriert und den Mandelsäureester aus dem organischen Filtrat durch Destillation isoliert.

2. Verfahren nach Anspruch 1, dadurch gekennzeichnet, daß man bei Temperaturen zwischen 45 und 100°C arbeitet.

3. Verfahren nach Anspruch 1, dadurch gekennzeichnet, daß man pro Mol Mandelsäurenitril der Formel (II) 5 bis 16 Mol eines Alkohols der Formel (III) einsetzt.

4. Verfahren nach Anspruch 1, dadurch gekennzeichnet, daß man pro Mol Mandelsäurenitril (II) 2,1 bis 2,5 Mol Chlorwasserstoff einsetzt.

5. Verfahren nach Anspruch 1, dadurch gekennzeichnet, daß man pro Mol Mandelsäurenitril (II) bis zu 1 Mol Wasser und 1,3 bis 2,2 Mol Chlorwasserstoff einsetzt.

6. Verfahren nach Anspruch 1, dadurch gekennzeichnet, daß man als Ausgangsstoff der Formel (II) Mandelsäurenitril und als Alkohol der Formel (III) Methanol oder Äthanol einsetzt.

7. Verfahren nach Anspruch 1, dadurch gekennzeichnet, daß man als aromatischen, gegebenenfalls chlorierten Kohlenwasserstoff Benzol, Toluol, Cumol, Xylol, Chlorbenzol, Dichlorbenzol oder Chlortoluol einsetzt.

8. Verfahren nach Anspruch 1, dadurch gekennzeichnet, daß man auf 1 Gew.-Teil Mandelsäurenitril 1 bis 20 Gew.-Teile, vorzugsweise 2 bis 15 Gew.-Teile des aromatischen, gegebenenfalls chlorierten Kohlenwasserstoffs einsetzt.

9. Verfahren gemäß Anspruch 1, dadurch gekennzeichnet, daß man gasförmiges Ammoniak bis zu einem pH-Wert zwischen 3 und 4, vorzugsweise zwischen 3,3 und 3,9, zufügt.

0006539

Nummer der Anmeldung

Europäisches Patentamt

# EUROPÄISCHER RECHERCHENBERICHT

EP 79 101 940.9

| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| A | DE - C - 562 390 (I.G. FARBEN)<br>* Beispiel 1 *<br>-- | 1 | C 07 C 69/66<br>C 07 C 67/22 |
| A | Chemical Abstracts, Band 45, Nr. 3, 10. Februar 1951<br>COLUMBUS,OHIO, USA<br>H.R. HENZE et al. "Synthesis of 5-benzhydryl-5-substituted hydantoins"<br>Spalte 1032 DE<br>& Journal of Organic Chemistry Band 15, 1950<br>Seiten 901 bis 907<br>-- | 1 | **RECHERCHIERTE SACHGEBIETE (Int. Cl.3)**<br>C 07 C 67/22<br>C 07 C 69/66<br>C 07 C 69/76 |
| D | Chemisches Zentralblatt, Band 109, Nr.15, 13. April 1938, Teil I,<br>Berlin<br>F. ADICKES et al. "Über die Herstellung einiger Säureester"<br>Seiten 3032 bis 3033<br>& Journal für praktische Chemie (NF) Band 150, 1938<br>Seiten 81 bis 93<br>-- ./.. | 1,5,6 | |

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 25-09-1979 | KNAACK |

Europäisches Patentamt

# EUROPÄISCHER RECHERCHENBERICHT

Nummer der Anmeldung
EP 79 101 940.9



| EINSCHLÄGIGE DOKUMENTE | | | KLASSIFIKATION DER ANMELDUNG (Int.Cl.3) |
|---|---|---|---|
| Kategorie | Kennzeichnung des Dokuments mit Angabe, soweit erforderlich, der maßgeblichen Teile | betrifft Anspruch | |
| A | Chemisches Zentralblatt, Band 97, Nr. 16, 21. April 1926, Teil I, Berlin, P. NICOLLE "Über einige acyclische trisubstituierte α -Glykole" Seiten 2683 bis 2684 & Bull. Soc. Chim. de France, Band 39, Seiten 55 bis 67 | 1 | |
| | -- | | RECHERCHIERTE SACHGEBIETE (Int. Cl.3) |
| A | Chemical Abstracts, Band 46, Nr. 3, 10. Ferbruar 1952, COLUMBUS, OHIO, USA H. GUDGEON et al. "Preparation of some substituted butadienes" Spalte 880 DE & J. Chem. Soc. Band 1951, Seiten 1926 bis 1928 | 1 | |
| | ---- | | |